# EUROPEAN PATENT APPLICATION

(11) **EP 4 796 529 A1**
(43) Date of publication of application: **26.08.2026**
(21) Application number: 25315061.9
(22) Date of filing: 21.02.2025
(51) Int. Cl.: C07C 303/22, C07C 309/86, C07C 303/38, C07C 311/16, C07C 303/40, C07C 311/48, H01M 10/052

(54) **MONOMER FOR SINGLE-ION BATTERY AND METHOD FOR SYNTHESISING SAME**

(71) Applicant: Blue Solutions, 29500 Ergué Gabéric (FR); Nantes Université, 44035 Nantes Cedex 1 (FR); Centre National de la Recherche Scientifique, 75794 Paris (FR)
(72) Inventor: Grenz, David, 29500 Ergué-Gabéric (FR); Neron, Sebastien, 29500 Ergué-Gabéric (FR); Yang, Jin, 29500 Ergué-Gabéric (FR); Degras, Sarah, 29500 Ergué-Gabéric (FR); Poizot, Philippe, 44035 Nantes Cedex 1 (FR)
(74) Representative: BCF Global

(57) **Abstract**

The present technology relates to methods for the synthesis of a PFAS-free lithium single-ion polymer which comprises simultaneously reacting a sulfonyl chloride compound with an aromatic sulfonamide compound and a compound that is suitable to act as a quenching base. The simultaneous reaction of sulfonyl chloride with the aromatic sulfonamide compound and the compound that is suitable to act as a quenching base yields the single-ion monomer.

## Description

### CROSS-REFERENCE TO RELATED APPLICATION

None.

### TECHNICAL FIELD

The present technology generally relates to lithium single-ion perfluoroalkyl and polyfluoroalkyl substances (PFAS)-free compounds, to methods for the synthesis of lithium single-ion PFAS-free polymer electrolytes, and to methods for the synthesis of lithium single-ion PFAS-free monomers.

### BACKGROUND

A lithium battery using a lithium metal as a negative electrode has excellent energy density. However, with repeated cycles, such a battery can be subject to dendrites' growths on the surface of the lithium metal electrode when recharging the battery as the lithium ions are unevenly re-plated on the surface of the lithium metal electrode. To minimize the effect of the morphological evolution of the surface of the lithium metal anode including dendrites growth, a lithium metal battery typically uses a pressure system and a solid polymer electrolyte adapted to resist the pressure applied thereto as described in U.S. Pat. No. 6,007,935, incorporated herein by reference. Over numerous cycles, dendrites on the surface of the lithium metal anode, however, may still grow to penetrate the solid polymer electrolyte, and eventually cause 'soft' short circuits between the negative electrode and the positive electrode, resulting in decreasing or poor performance of the battery. Therefore, the growth of dendrites may still deteriorate the cycling characteristics of the battery and constitutes a major limitation with respect to the optimization of the performance of lithium batteries having a metallic lithium anode.

Various types of solid polymer electrolytes adapted for use with lithium metal electrodes have been developed since the late 1970s to overcome this issue but have been found to lack in conductivity and/or mechanical properties. Single-ion-conducting polymer electrolytes have, however, emerged as promising candidates, as the transference number of lithium cation approaches unity, and therefore prevents the formation of concentration gradients across the electrolyte, and dendrite formation as a result.

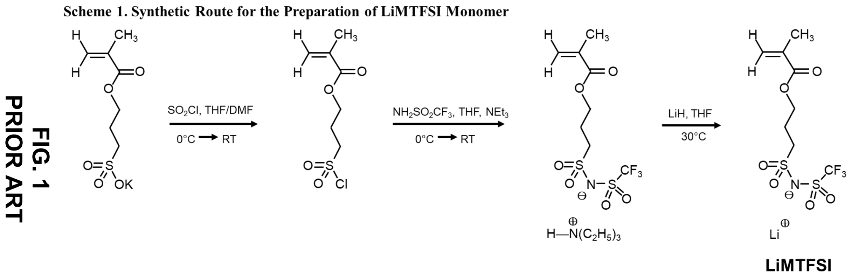

One of the best-known solid polymer electrolytes is Lithium Polystyrene trifluorosulfonimide Li(PSTFSI). Existing methods of synthesis of Li(PSTFSI) comprise at least 3 steps (as represented in FIG. 1, and disclosed in ACS Appl. Mater. Interfaces 2016, 8, 10350-10359, incorporated herein by reference). A typical synthesis generally starts from a vinyl monomer (acrylate, methacrylate, styrene, etc.) bearing a sulfonate end group. The sulfonate is first converted into sulfonyl chloride using either thionyl chloride or oxalyl chloride. In the second step, trifluoromethanesulfonamide (NH₂SO₂CF₃) is attached to generate the (trifluoromethylsulfonyl)imide (TFSI) anion. In this step, as seen in FIG. 1, triethylamine (TEA) is used as a base to quench the byproduct HCl and acidic protons on the TFSI anions, resulting in a nitrogen-based organic cation, or more specifically, a triethylammonium cation, on the intermediate monomer. Finally, in the third step triethylammonium cations are substituted by lithium cations using LiH. The final product is then generally isolated by recrystallization (step not shown).

Other methods have also included exchanging the triethylammonium cations obtained by the methods disclosed above, with potassium cations, and then exchanging the potassium cation for a lithium cation in additional steps either before or after cross-linkage of the monomers (e.g., Polym. Chem., 2016, 7, 6901-6910; Journal of Polymer Science, 2020, 58, 2376-2388; and Electrochimica Acta, 2011, 57, 14- 19, all incorporated herein by reference).

The methods disclosed above, however, suffer from several disadvantages. Existing methods generally use LiH in the ion-exchange step, which can be hazardous to handle in large quantities. In addition, the polymer obtained is considered as a perfluoroalkyl and polyfluoroalkyl substances (PFAS) containing-compound. Perfluoroalkyl and polyfluoroalkyl substances (PFAS) are increasingly of interest to environmental regulators in Europe, and abroad. While they show many advantages, they are also bioaccumulative and environmentally persistent, as they don't degrade under normal conditions. Environmental and health regulators have been concerned about the effects of exposure to PFAS on human health and the environment. In addition to their toxicity, these compounds tend to be expensive due to their complex synthesis limiting the application of their derivative polymers. Therefore, there is a need for alternative lithium single-ion polymers which match de the electrochemical performances while overcoming at least some of the above-described problems.

### SUMMARY

From a broad aspect, the present technology relates to methods of synthesis of lithium single-ion polymers which are PFAS-free.

From one aspect there is provided a method for the synthesis of a lithium single-ion monomer which comprises simultaneously reacting a sulfonyl chloride compound with: i) an aromatic sulfonamide compound; and ii) a compound that is suitable to act as a quenching base. The simultaneous reaction of sulfonyl chloride with the aromatic sulfonamide compound and the compound that is suitable to act as a quenching base yields the single-ion monomer.

From another aspect, the present technology provides for a method for the synthesis of a PFAS-free lithium single-ion polymer, the method comprising simultaneously reacting a sulfonyl chloride compound with: i) a n aromatic sulfonamide compound; and ii) a compound that is suitable to act as a quenching base; wherein the simultaneous reaction of sulfonyl chloride with the aromatic sulfonamide compound and the compound that is suitable to act as a quenching base yields the single-ion monomer; wherein the sulfonyl chloride compound is of formula: 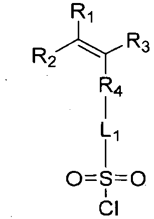 wherein: R₁ and R₂ are each independently H or F; R₃ is H, F, CN or CH₃; R₄ is an ester group, a phenyl group with L₁ substituted at ortho, para, or meta position, a fully or partially fluorinated phenyl group with L₁ substituted at ortho, para or meta position, an amide group, a carbonate group, or an ether group; and L₁ is a n-alkyl group (e.g., n=1-16), a fluorinated alkyl group (e.g., C₁-C₁₆), a branched alkyl group (e.g., C₃-C₁₆), an ethylene oxide linker (e.g., C₄ to C₁₆), a fluorinated ethylene oxide linker (e.g., C₄ to C₁₆), a cycloalkyl group (e.g., C₄ to C₇), a fluorinated cycloalkyl group (e.g., C₄ to C₇), or L₁ is absent. From another aspect, there is provided a method for the synthesis of a lithium single-ion monomer which comprises: 1) obtaining a sulfonyl chloride compound from a sulfonate; 2) simultaneously reacting a sulfonyl chloride compound with: i) an aromatic sulfonamide; and ii) a compound that is suitable to act as a quenching base to obtain unpurified lithium single-ion monomer; and 3) purifying the unpurified lithium single-ion monomer.

From another aspect, the methods of the present technology are safer to carry out compared to existing methods as the bases used in synthesis are safer to handle in scale-up production.

From another aspect the methods of the present technology result in a final product which is substantially free of impurities.

From a broad aspect, the present technology relates to lithium single-ion compounds which are PFAS-free.

From a broad aspect, the present technology relates to methods of synthesis of sulfonimide lithium single-ion polymers which are PFAS-free.

From a broad aspect, the present technology relates to a method for the synthesis of a lithium single-ion perfluoroalkyl and polyfluoroalkyl substances (PFAS)-free monomer, the method comprising simultaneously reacting a sulfonyl chloride compound with: i) an aromatic sulfonamide compound; and ii) a compound that is suitable to act as a quenching base; wherein the simultaneous reaction of sulfonyl chloride with the aromatic sulfonamide compound and the compound that is suitable to act as a quenching base yields the single-ion monomer; wherein the sulfonyl chloride compound is of formula: 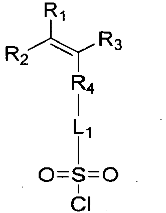 wherein R₁ and R₂ are each independently H or F; R₃ is H, F, CN or CH₃; R₄ is an ester group, a phenyl group with L₁ substituted at ortho, para, or meta position, a fully or partially fluorinated phenyl group with L₁ substituted at ortho, para or meta position, an amide group, a carbonate group, or an ether group; and L₁ is a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent.

From a broad aspect, the present technology relates to a method as defined herein, wherein the aromatic sulfonamide compound has a formula R_{A}-SO₂-NH₂, wherein R_{A} is a substituted aryl compound.

From a broad aspect, the present technology relates to a method as defined herein, wherein the substituted aryl compound comprises an electron withdrawing group.

From a broad aspect, the present technology relates to a method as defined herein, wherein the electron withdrawing group is selected from -CN, -NO₂, -F, and -OMe.

From a broad aspect, the present technology relates to a method as defined herein, wherein the sulfonate compound is of formula: 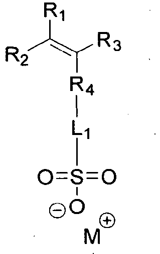 wherein R₁ and R₂ are each independently H or F; R₃ is H, F, CN or CH₃; R₄ is an ester group, a phenyl group with L₁ substituted at ortho, para, or meta position, a fully or partially fluorinated phenyl group with L₁ substituted at ortho, para, or meta position, an amide group, a carbonate group, or an ether group; L₁ is a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent; and M is monovalent cation H⁺, Li⁺, Na⁺, K⁺, Rb⁺ or Cs⁺.

From a broad aspect, the present technology relates to a method as defined herein, wherein the conversion from sulfonate to sulfonyl chloride is performed using one or more of thionyl chloride and oxalyl chloride.

From a broad aspect, the present technology relates to a method as defined herein, wherein the sulfonyl chloride compound has the formula: 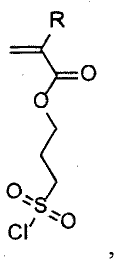 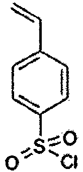 wherein R is H or CH3, , or

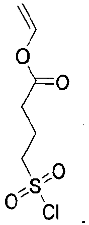

From a broad aspect, the present technology relates to a method as defined herein, wherein the sulfonate has the formula: 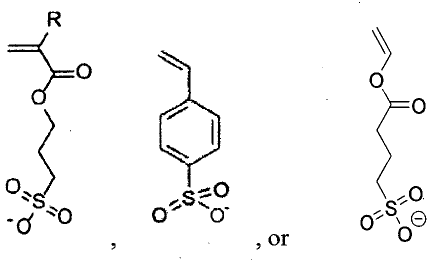 wherein R is H or CH₃.

From a broad aspect, the present technology relates to a method for the synthesis of a lithium single-ion perfluoroalkyl and polyfluoroalkyl substances (PFAS)-free monomer, the method comprising simultaneously reacting a sulfonyl chloride compound with: i) an aromatic sulfonamide compound; and ii) a compound that is suitable to act as a quenching base; wherein the simultaneous reaction of sulfonyl chloride with the aromatic sulfonamide compound and the compound that is suitable to act as a quenching base yields the single-ion monomer; wherein the aromatic sulfonamide compound is of formula: 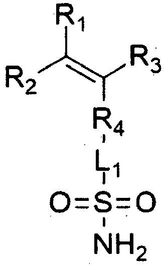 wherein R₁ and R₂ are each independently H or F; R₃ is H, F, CN or CH₃; R₄ is an ester group, a phenyl group with L₁ substituted at ortho, para, or meta position, a fully or partially fluorinated phenyl group with L₁ substituted at ortho, para or meta position, an amide group, a carbonate group, or an ether group; and L₁ is a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent.

From a broad aspect, the present technology relates to a method as defined herein, wherein the sulfonyl chloride compound has a formula R_{A}-SO₂-Cl, wherein R_{A} is a substituted aryl compound.

From a broad aspect, the present technology relates to a method as defined herein, wherein the substituted aryl compound comprises an electron withdrawing group.

From a broad aspect, the present technology relates to a method as defined herein, wherein the electron withdrawing group is selected from -CN, -NO₂, -F, and -OMe.

From a broad aspect, the present technology relates to a method as defined herein, wherein the compound that is suitable to act as a quenching base is a lithium-containing basic compound.

From a broad aspect, the present technology relates to a method as defined herein, wherein the sulfonate compound is of formula: 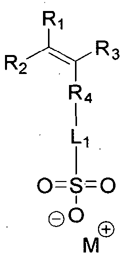 wherein R1 and R2 are each independently H or F; R3 is H, F, CN or CH3; R4 is an ester group, a phenyl group with L1 substituted at ortho, para, or meta position, a fully or partially fluorinated phenyl group with L1 substituted at ortho, para, or meta position, an amide group, a carbonate group, or an ether group; L1 is a n-alkyl group, a fluorinated alkyl group, a branched alkyl group, an ethylene oxide linker, a fluorinated ethylene oxide linker, a cycloalkyl group, a fluorinated cycloalkyl group, or L1 is absent; and M is monovalent cation H+, Li+, Na+, K+, Rb+ or Cs+.

From a broad aspect, the present technology relates to a method as defined herein, wherein the lithium single-ion (PFAS)-free monomer has formula: 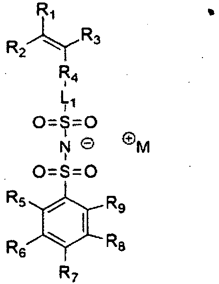 wherein R₁ and R₂ are independently H or F; R₃ is H, F, CN or CH₃; R₄ is an ester group, a phenyl group with L1 substituted at ortho, para, or meta position, a fully or partially fluorinated phenyl group with L₁ substituted at ortho, para, or meta position, an amide group, a carbonate group, or an ether group; L₁ can be a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent; R₅, R₆, R₇, R₈, R₉ are selected from H, F, CN, NO₂, an alkoxy group; and M is monovalent cation H⁺, Li⁺, Na⁺, K⁺, Rb⁺ or Cs⁺.

From a broad aspect, the present technology relates to a method as defined herein, further comprising polymerizing the lithium single-ion (PFAS)-free monomer.

From a broad aspect, the present technology relates to a method as defined herein, wherein the method comprises the following steps: 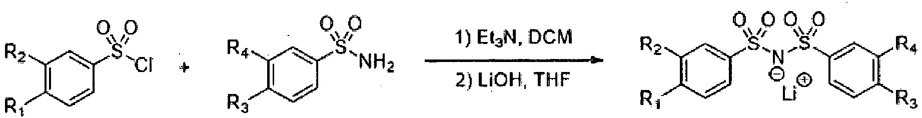 wherein R1, R2, R3 and R4 are independently H, CH₃, C₂H₃, F, OMe or NO₂.

From a broad aspect, the present technology relates to a method as defined herein, wherein the lithium single-ion (PFAS)-free compound is selected from:
i) Poly(lithium (phenylsulfonyl)((4-vinylphenyl)sulfonyl)amide) (PSSI - Li),
ii) Poly(lithium (4-nitrophenyl)sulfonyl)((4-vinylphenyl)sulfonyl)amide) (NPSSI - Li),
iii) Poly(lithium (4-fluorophenyl)sulfonyl)((4-vinylphenyl)sulfonyl)amide) (FPSSI - Li),
iv) Poly(lithium (3,4-difluorophenyl)sulfonyl)((4-vinylphenyl)sulfonyl)amide)(F2PSSI - Li),
v) Poly(lithium (3,4-dinitrophenyl)sulfonyl)((4-vinylphenyl)sulfonyl)amide) (N2PSSI - Li)
vi) Poly(lithium difluorovinyl diphenyl sulfonimide) (PPSVSA-F2),
vii) Lithium (4-fluorophenyl)(4'-styrenyl)disulfonimide (FPSSI·Li),
viii) p(FPSSi·Li),
ix) Lithium (4-anisyl)(4'-styrenyl)disulfonimide (ASSI·Li),
x) Lithium (4-acetamidophenyl)(4'-styrenyl)disulfonimide (AAPSSI·Li),
xi) Lithium (4-phenyl)(4'-styrenyl)disulfonimide (PSSI·Li),
xii) p(PSSI-Li),
xiii) Lithium (4-nitrophenyl)(4'-styrenyl)disulfonimide (NPSSI·Li),
xiv) p(NPSSI·Li),
xv) Lithium (3,4-difluorophenyl)(4'-styrenyl)disulfonimide (F2PSSI·Li),
xvi) p(F2PSSI·Li),
xvii) Lithium (4-fluorophenyl) (4'-styrenyl)disulfonimide (FPSSI·Li),
xviii) p(FPSSI·Li), Lithium (4-anisyl)(4'-styrenyl)disulfonimide (ASSI·Li), and
xix) Lithium (4-acetamidophenyl)(4'-styrenyl)disulfonimide (AAPSSI·Li).

From a broad aspect, the present technology relates to a method for the synthesis of a lithium single-ion monomer, the method comprising: 1) obtaining a sulfonyl chloride compound from a sulfonate; 2) simultaneously reacting the sulfonyl chloride compound with: i) an aromatic sulfonamide compound, and ii) a compound that is suitable to act as a quenching base to obtain unpurified lithium single-ion monomer; and 3) purifying the unpurified lithium single-ion (PFAS)-free monomer.

From a broad aspect, the present technology relates to a method as defined herein, wherein the aromatic sulfonamide compound has the formula R_{A}-SO2-NH2, wherein R_{A} is a substituted aryl compound.

From a broad aspect, the present technology relates to a method as defined herein,, wherein the substituted aryl compound comprises an electron withdrawing group.

From a broad aspect, the present technology relates to a method as defined herein,, wherein the electron withdrawing group is selected from -CN, -NO₂, -F and -OMe.

From a broad aspect, the present technology relates to a method as defined herein,, wherein the lithium single-ion (PFAS)-free compound is selected from:
i) Poly(lithium (phenylsulfonyl)((4-vinylphenyl)sulfonyl)amide) (PSSI - Li),
ii) Poly(lithium (4-nitrophenyl)sulfonyl)((4-vinylphenyl)sulfonyl)amide) (NPSSI - Li),
iii) Poly(lithium (4-fluorophenyl)sulfonyl)((4-vinylphenyl)sulfonyl)amide) (FPSSI - Li),
iv) Poly(lithium (3,4-difluorophenyl)sulfonyl)((4-vinylphenyl)sulfonyl)amide)(F2PSSI - Li),
v) Poly(lithium (3,4-dinitrophenyl)sulfonyl)((4-vinylphenyl)sulfonyl)amide) (N2PSSI - Li)
vi) Poly(lithium difluorovinyl diphenyl sulfonimide) (PPSVSA-F2),
vii) Lithium (4-fluorophenyl)(4'-styrenyl)disulfonimide (FPSSI·Li),
viii) p(FPSSi·Li),
ix) Lithium (4-anisyl)(4'-styrenyl)disulfonimide (ASSI·Li),
x) Lithium (4-acetamidophenyl)(4'-styrenyl)disulfonimide (AAPSSI·Li),
xi) Lithium (4-phenyl)(4'-styrenyl)disulfonimide (PSSI·Li),
xii) p(PSSI·Li),
xiii) Lithium (4-nitrophenyl)(4'-styrenyl)disulfonimide (NPSSI·Li),
xiv) p(NPSSI·Li),
xv) Lithium (3,4-difluorophenyl)(4'-styrenyl)disulfonimide (F2PSSI·Li),
xvi) p(F2PSSI·Li),
xvii) Lithium (4-fluorophenyl) (4'-styrenyl)disulfonimide (FPSSI·Li),
xviii) p(FPSSI·Li), Lithium (4-anisyl)(4'-styrenyl)disulfonimide (ASSI·Li), and
xix) Lithium (4-acetamidophenyl)(4'-styrenyl)disulfonimide (AAPSSI·Li).

From a broad aspect, the present technology relates to a lithium single-ion (PFAS)-free monomer having formula: 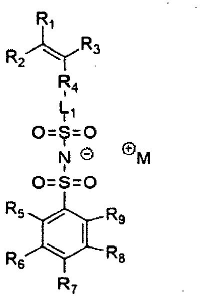 wherein R₁ and R₂ are independently H or F; R₃ is H, F, CN or CH₃; R₄ is an ester group, a phenyl group with L₁ substituted at ortho, para, or meta position, a fully or partially fluorinated phenyl group with L₁ substituted at ortho, para, or meta position, an amide group, a carbonate group, or an ether group; L₁ can be a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent; R₅, R₆, R₇, R₈, R₉ are selected from H, F, CN, NO₂, an alkoxy group; and M is monovalent cation H⁺, Li⁺, Na⁺, K⁺, Rb⁺ or Cs⁺.

From a broad aspect, the present technology relates to ahe lithium single-ion (PFAS)-free monomer as defined herein, being a sulfonimide lithium single-ion (PFAS)-free monomer.

From a broad aspect, the present technology relates to a lithium single-ion (PFAS)-free compound selected from:
i) Poly(lithium (phenylsulfonyl)((4-vinylphenyl)sulfonyl)amide) (PSSI - Li),
ii) Poly(lithium (4-nitrophenyl)sulfonyl)((4-vinylphenyl)sulfonyl)amide) (NPSSI - Li),
iii) Poly(lithium (4-fluorophenyl)sulfonyl)((4-vinylphenyl)sulfonyl)amide) (FPSSI - Li),
iv) Poly(lithium (3,4-difluorophenyl)sulfonyl)((4-vinylphenyl)sulfonyl)amide)(F2PSSI - Li),
v) Poly(lithium (3,4-dinitrophenyl)sulfonyl)((4-vinylphenyl)sulfonyl)amide) (N2PSSI - Li)
vi) Poly(lithium difluorovinyl diphenyl sulfonimide) (PPSVSA-F2),
vii) Lithium (4-fluorophenyl)(4'-styrenyl)disulfonimide (FPSSI·Li),
viii) p(FPSSi·Li),
ix) Lithium (4-anisyl)(4'-styrenyl)disulfonimide (ASSI·Li),
x) Lithium (4-acetamidophenyl)(4'-styrenyl)disulfonimide (AAPSSI·Li),
xi) Lithium (4-phenyl)(4'-styrenyl)disulfonimide (PSSI·Li),
xii) p(PSSI·Li),
xiii) Lithium (4-nitrophenyl)(4'-styrenyl)disulfonimide (NPSSI-Li),
xiv) p(NPSSI·Li),
xv) Lithium (3,4-difluorophenyl)(4'-styrenyl)disulfonimide (F2PSSI·Li),
xvi) p(F2PSSI·Li),
xvii) Lithium (4-fluorophenyl) (4'-styrenyl)disulfonimide (FPSSI·Li),
xviii) p(FPSSI·Li), Lithium (4-anisyl)(4'-styrenyl)disulfonimide (ASSI·Li), and
xix) Lithium (4-acetamidophenyl)(4'-styrenyl)disulfonimide (AAPSSI·Li).

### BRIEF DESCRIPTION OF THE DRAWING

Reference will now be made to the accompanying drawing.

FIG. 1 illustrates a typical 3-step synthesis of a lithium single-ion monomer according to Procarreli et al., ACS Appl. Mater. Interfaces 2016, 8, 10350-10359 (incorporated herein by reference).

### DETAILED DESCRIPTION

### Definition

The use of "including", "comprising", or "having", "containing", "involving" and variations thereof herein, is meant to encompass the items listed thereafter as well as, optionally, additional items.

It must be noted that, as used in this specification and the appended claims, the singular form "a", "an" and "the" include plural referents unless the context clearly dictates otherwise.

As used herein, the term "and/or" is to be taken as specific disclosure of each of the two specified features or components with or without the other. For example, "A and/or B" is to be taken as specific disclosure of each of (i) A, (ii) B and (iii) A and B, just as if each is set out individually herein.

As used herein, the term "about" in the context of a given value or range refers to a value or range that is within 20%, preferably within 10%, and more preferably within 5% of the given value or range.

As used herein the term "substantially" means to a great or significant extent.

As used herein, the expression "electron withdrawing group" refers to an atom or group that draws electron density from neighboring atoms towards itself, usually by resonance or inductive effects.

As used herein, the expressions "PFAS-free lithium single-ion compound", "PFAS-free lithium single-ion monomer" and the expression "PFAS-free lithium single-ion polymer" refer to a lithium single-ion compound, monomer, or polymer that is substantially free of or that are entirely free of perfluoroalkyl and polyfluoroalkyl substances. In some implementations, a PFAS-free lithium single-ion monomer and a PFAS-free lithium single-ion polymer are types of PFAS-free lithium single-ion compounds.

In one embodiment, the present technology relates to PFAS-free lithium single-ion compounds obtained by the methods disclosed herein. In some implementations, the PFAS-free lithium single-ion compounds obtained by the methods disclosed herein are PFAS-free polysulfonimide lithium single-ion compounds.

Examples of PFAS-free lithium single ion compounds include, but are not limited to: PSSI - Li Poly(lithium (phenylsulfonyl)((4-vinylphenyl)sulfonyl)amide), NPSSI - Li Poly(lithium (4-nitrophenyl)sulfonyl)((4-vinylphenyl)sulfonyl)amide), FPSSI - Li Poly(lithium (4-fluorophenyl)sulfonyl)((4-vinylphenyl)sulfonyl)amide), F2PSSI - Li Poly(lithium (3,4-difluorophenyl)sulfonyl)((4-vinylphenyl)sulfonyl)amide), Poly(lithium difluorovinyl diphenyl sulfonimide) (PPSVSA-F2), Lithium (4-fluorophenyl)(4'-styrenyl)disulfonimide (FPSSI·Li), p(FPSSi·Li), Lithium (4-anisyl)(4'-styrenyl)disulfonimide (ASSI·Li), Lithium (4-acetamidophenyl)(4'-styrenyl)disulfonimide (AAPSSI·Li), Lithium (4-phenyl)(4'-styrenyl)disulfonimide (PSSI·Li), p(PSSI·Li), Lithium (4-nitrophenyl)(4'-styrenyl)disulfonimide (NPSSI·Li), p(NPSSI·Li), Lithium (3,4-difluorophenyl)(4'-styrenyl)disulfonimide (F2PSSI·Li), p(F2PSSI·Li), Lithium (4-fluorophenyl) (4'-styrenyl)disulfonimide (FPSSI·Li), p(FPSSI·Li), Lithium (4-anisyl)(4'-styrenyl)disulfonimide (ASSI·Li), and Lithium (4-acetamidophenyl)(4'-styrenyl)disulfonimide (AAPSSI·Li). Examples of PFAS-free lithium single ion compounds are illustrated below:

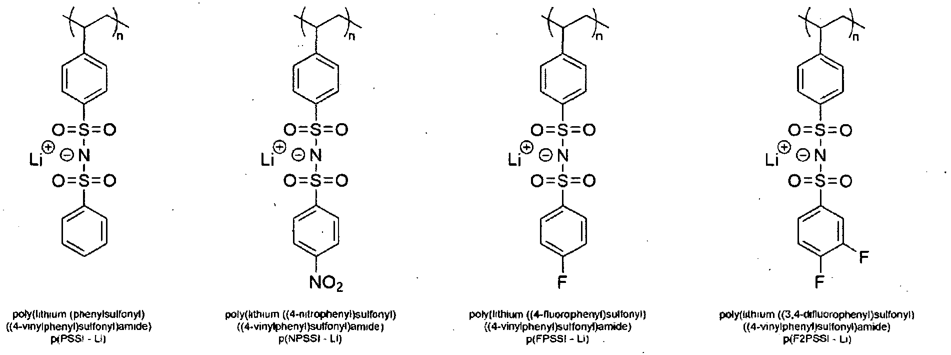

The present disclosure is not limited in its application to the details of construction and the arrangement of components set forth in the following description or illustrated in the drawings. The disclosure is capable of other embodiments and of being practiced or of being carried out in various ways. Also, the phraseology and terminology used herein is for the purpose of description and should not be regarded as limiting.

Broadly, the present technology provides for methods of synthesis of lithium single-ion polymers which are simpler, safer, less costly and more efficient than existing methods of synthesis of lithium single-ion monomers.

In certain embodiments, the methods of the present technology comprise simultaneously reacting a sulfonyl chloride compound with an aromatic sulfonamide compound leading to a PFAS-free single-ion monomer.

In some embodiments, the sulfonyl chloride compound has the following formula: 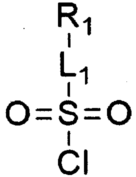 wherein: R₁ is H or F; R₃ is H, F, CN or CH₃; and L₁ is a n-alkyl group (e.g., n=1-16), a fluorinated alkyl group (e.g., C₁-C₁₆), a branched alkyl group (e.g., C₃-C₁₆), an ethylene oxide linker (e.g., C₄ to C₁₆), a fluorinated ethylene oxide linker (e.g., C₄ to C₁₆), a cycloalkyl group (e.g., C₄ to C₇), a fluorinated cycloalkyl group (e.g., C₄ to C₇), or L₁ is absent.

In some embodiments, the sulfonyl chloride compound has the following formula: 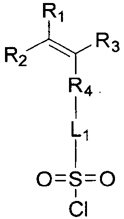 wherein: R₁ and R₂ are each independently H or F; R₃ is H, F, CN or CH₃; R₄ is an ester group, a phenyl group with L₁ substituted at ortho, para, or meta position, a fully or partially fluorinated phenyl group with L₁ substituted at ortho, para or meta position, an amide group, a carbonate group, or an ether group; and L₁ is a n-alkyl group (e.g., n=1-16), a fluorinated alkyl group (e.g., C₁-C₁₆), a branched alkyl group (e.g., C₃-C₁₆), an ethylene oxide linker (e.g., C₄ to C₁₆), a fluorinated ethylene oxide linker (e.g., C₄ to C₁₆), a cycloalkyl group (e.g., C₄ to C₇), a fluorinated cycloalkyl group (e.g., C₄ to C₇), or L₁ is absent.

In some instances, the ester group is -(C=O)-O- or -O-(C=O)-). In some instances, the amide group is -(C=O)-NH-. In some instances, the carbonate group is(-O-(C=O)-O-). In some instances, the ether group is (-O-).

In some embodiments, the aromatic sulfonamide compound has the formula R_{A}-SO₂-NH₂, wherein R_{A} is an aryl compound. In some instances, the substituted aryl compound comprises an electron-withdrawing group.

As used herein, the expression "aryl compound" refers to functional group or substituent derived from an aromatic ring.

In some embodiments the electron-withdrawing group is selected from -CN, - NO₂, -F, and -OMe.

In certain embodiments, as will be shown below, the aromatic sulfonamide compound is benzesulfonamide.

In certain embodiments, the compound that is suitable to act as a quenching base is a basic compound. In some embodiments, the basic compound may be selected from triethylamine, K₂CO₃, Na₂CO₃, Li₂CO₃, LiOH, Li₂SO₃, Li₃PO₄, lithium acetate, and lithium formate, and combinations thereof. In one embodiment, the basic compound is triethylamine.

In certain embodiments, the aromatic sulfonamide compound and the compound that is suitable to act as a quenching base are dissolved in an anhydrous solvent and mixed together prior to the addition of the sulfonyl chloride compound. In some embodiments, the anhydrous solvent may be selected from anhydrous methyl cyanide (MeCN), tetrahydrofuran (THF), acetone, dimethylformamide (DMF), and dimethyl sulfoxide (DMSO). Aqueous solvents such as water, alcohol and amine-containing solvents are not considered suitable for the methods of the present technology as water and hydroxy groups can react with the sulfonyl chloride to produce sulfonic acid, which is not the desired product. Therefore, in certain embodiments, the aromatic sulfonamide compound and the compound that is suitable to act as a quenching base are not dissolved in an aqueous solvent.

In certain embodiments, the aromatic sulfonamide compound and the compound that is suitable to act as a quenching base are mixed together at a temperature of from between about 15°C and about 30°C. In other embodiments, the aromatic sulfonamide compound and the compound that is suitable to act as a quenching base are mixed together at a temperature of about 15°C, about 20°C, about 25°C (i.e., room temperature (RT)), or about 30°C. In some embodiments, the aromatic sulfonamide compound and the compound that is suitable to act as a quenching base are mixed together at a temperature of about 25°C (RT).

In other embodiments, the aromatic sulfonamide compound and the compound that is suitable to act as a quenching base are mixed together from about 30 minutes to about 2 hours. In other embodiments, the aromatic sulfonamide compound and the compound that is suitable to act as a quenching base are mixed together for about 30 minutes, for about 45 minutes, for about 1 hour, for about 1.5 hours, or for about 2 hours. In some embodiments, the aromatic sulfonamide compound and the compound that is suitable to act as a quenching base are mixed together for about 1 hour.

In certain embodiments, the mixture of the aromatic sulfonamide compound and the compound that is suitable to act as a quenching base is cooled to a temperature of from about -5°C and about +4°C prior to the addition of the sulfonyl chloride compound.

In some embodiments, the mixture of the aromatic sulfonamide compound and the compound that is suitable to act as a quenching base is cooled to a temperature of about 0°C. The temperature of the mixture can be cooled to from about - 5°C to about + 4°C, or to about 0°C, using known techniques such as an ice-water bath or the like.

In some embodiments, the sulfonyl chloride compound may also be dissolved in an anhydrous solvent prior to its addition to the mixture of the aromatic sulfonamide compound and the compound that is suitable to act as a quenching base. In certain embodiments, the anhydrous solvent is the same as the anhydrous solvent used to dissolve the aromatic sulfonamide compound and the compound that is suitable to act as a quenching base. In other embodiments, the anhydrous solvent in which the sulfonyl chloride is dissolved is different than the anhydrous solvent used to dissolve the aromatic sulfonamide compound and the compound that is suitable to act as a quenching base. In such embodiments, the two different solvents are miscible in one another. In some embodiments, the sulfonyl chloride compound is dissolved in an anhydrous solvent selected from anhydrous MeCN, THF, acetone, DMF, and DMSO. For the same reasons provided above, the sulfonyl chloride compound is not dissolved in an aqueous solvent.

In certain embodiments, the mixture of the sulfonyl chloride and the anhydrous solvent is added to the mixture of the aromatic sulfonamide compound and the compound that is suitable to act as a quenching base slowly and /or in a dropwise fashion. This prevents temperature jumps and solvent evaporation, and allows for the HCl by-product to be quenched efficiently by the quenching base.

The methods of the present technology, however, are not limited to a particular order in which the reagents are added. Therefore, in other embodiments, the mixture of the aromatic sulfonamide compound and the compound that is suitable to act as a quenching base dissolved in the anhydrous solvent may be added to the sulfonyl chloride compound previously dissolved in an anhydrous solvent.

In other embodiments, the reaction of the sulfonyl chloride with the aromatic sulfonamide and the compound that is suitable to act as a quenching base is carried out at a starting temperature of from about -5°C and brought up to about +30°C. In some embodiments, said reaction is carried out at a starting temperature of from about 0°C and brought up to about 25°C. In other embodiments, said reaction is carried out for about 30 minutes to about 5 hours. In further embodiments, said reaction is carried out for about 30 minutes to about 1 hour, about 30 minutes to about 1.5 hours, about 1.5 hours to about 3.5 hours, about 2 hours to about 3 hours, or about 2 hours to about 4 hours. In some embodiments, said reaction is carried out for about 2.5 hours. In another embodiment, said reaction is carried out for about 3 hours. In yet another embodiment, said reaction is carried out for about 30 minutes. In further embodiments, said reaction is carried out at a starting temperature of about 0°C and brought up to about 25°C for about 2.5 hours. In other embodiments, said reaction is carried out at a starting temperature of about 0°C and brought up to about 25°C for about 3 hours. In yet other embodiments, said reaction is carried out at a starting temperature of about 0°C and brought up to about 25°C for about 30 minutes.

In some embodiments, the sulfonyl chloride compound has formula: 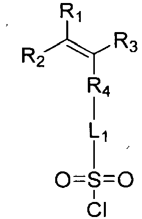 wherein R₁ and R₂ are each independently H or F; R₃ is H, F, CN or CH₃; R₄ is an ester group, a phenyl group with L₁ substituted at ortho, para, or meta position, a fully or partially fluorinated phenyl group with L₁ substituted at ortho, para, or meta position, an amide group, a carbonate group, or an ether group; L₁ is a n-alkyl group (e.g., n=1-16), a fluorinated alkyl group (e.g., C₁-C₁₆), a branched alkyl group (e.g., C₃-C₁₆), an ethylene oxide linker (e.g., C₄ to C₁₆), a fluorinated ethylene oxide linker (e.g., C₄ to C₁₆), a cycloalkyl group (e.g., C₄ to C₇), a fluorinated cycloalkyl group (e.g., C₄ to C₇), or L₁ is absent.

In some instances, the ester group is -(C=O)-O- or -O-(C=O)-). In some instances, the amide group is -(C=O)-NH-. In some instances, the carbonate group is (-O-(C=O)-O-). In some instances, the ether group is (-O-).

In certain embodiments, the sulfonyl chloride compound is converted from a sulfonate.

In certain embodiments, the sulfonate comprises a vinyl monomer such as acrylate, methacrylate, styrene or vinyl acetate. In other embodiments, the sulfonate has the formula: 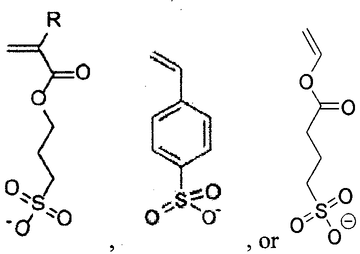 wherein R is H or CH₃.

In certain embodiments the cation associated with the sulfonate may be a monovalent cation selected from H⁺, K⁺, Na⁺, Li⁺, Rb⁺, and Cs⁺.

In certain embodiments, the sulfonyl chloride has the formula : 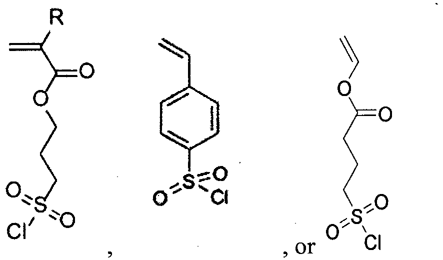 wherein R is H or CH₃.

In some embodiments, the conversion of the sulfonate to sulfonyl chloride is performed using one or more of thionyl chloride and oxalyl chloride. In certain embodiments, said conversion is carried out at a starting temperature of about -5°C and brought up to about +30°C. In some embodiments, said conversion is carried out at a starting temperature of about 0°C and brought up to about 25°C. In other embodiments, said conversion is carried out for about 12 hour to about 36 hours. In further embodiments, said conversion is carried out for about 14 hours to about 18 hours, about 20 hours to about 26 hours. In some embodiments, said conversion is carried out for about 16 hours. In other embodiments, said conversion is carried out for about 24 hours. In other embodiments, said conversion is carried out at a starting temperature of about 0°C and brought up to about 25°C for about 16 hours. In further embodiments, said conversion is carried out at a starting temperature of about 0°C and brought up to about 25°C for about 24 hours.

In certain embodiments, the methods of the present technology further comprise a step of purifying the lithium single-ion monomer. In certain implementations of these embodiments, the step of purifying the lithium single-ion monomer includes purifying by silica gel flash chromatography or by recrystallization.

In some embodiments, the lithium single-ion monomer is purified by recrystallization.

In other embodiments, inhibitors preventing self-polymerization may be used in the step of purifying the lithium single-ion monomer. In certain embodiments, the inhibitors may be added to the column elution fractions that contain the pure product during silica gel flash chromatography. In such embodiments, the elution solvent may be removed by rotavap, leaving monomers well mixed with the inhibitors.

Inhibitors suitable for the methods of the present technology include 4-methoxyphenol (also referred to as MEHQ) and butylated hydroxytoluene (BHT). In some embodiments, the inhibitors may be used at ppm levels including from about 100 ppm to about 500 ppm. In some embodiments, about 3 mg to about 5mg of MEHQ may be added to about 20g of product to prevent self-polymerization.

In other embodiments, the step of purifying the lithium single-ion monomer comprises removing the LiCl byproduct before purifying by silica gel flash chromatography. In certain implementations of these embodiments, the step of removing the LiCl includes filtering the reaction product before running the silica gel flash chromatography.

In other embodiments, the methods of the present technology further comprise polymerizing the lithium single-ion monomer to obtain a lithium single-ion polymer. In certain embodiments polymerization may be performed by controlled polymerization (ATRP ("Atom Transfer Radical Polymerization"), RAFT ("Reversible Addition Fragmentation Chain Transfer"), anionic polymerization, cationic polymerization, free radical polymerization, or NMP ("Nitroxide-Mediated Radical Polymerization")). In the methods of the present technology, polymerization of the final lithium single-ion monomer resulted in a white powder with no colored impurities.

In certain embodiments, the methods of the present technology comprise the following steps: 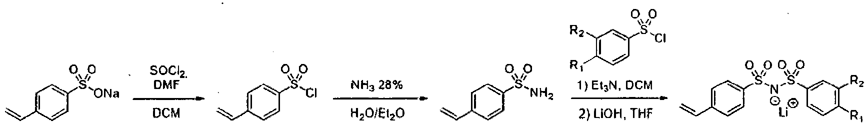 wherein R₁ and R₂ are independently H, F, Cl, Br, CH₃, I, OH, OMe, COOH, COOR, CHO, COCl, COF, CN, CH(COR)₂, CH(CN)₂, OAc, OTMS, OTBS, OC(CH₃)₃, O(CO)OC(CH₃)₃, O(CH₂CH₂O)ₙ, O(CH(CH₃)CH₂O)ₙ, OSO₃H, OSO₃R, OSO₂F, OSO₂Cl, OSO(NR)F, NO₂, NH₂, NHR, NR₂, NHAc, NH(CO)OC(CH₃)₃ NSOF₂, NSO(OR)F, NSO(NR)F, NSO(OR)₂, NSO(NR)₂, NCO, SH, SR, SO₃H, SO₂F, SO₂Cl, SO₂NH₂, SO₂NHR, SO₂NR₂, SO₂(OR) or SO₂NHSO₂R, wherein R is an alkyl compound.

In certain embodiments, the methods of the present technology comprise the first step synthesis in an aprotic solvent such as DCM, CHCl₃, CCl₄, Cl₂C=CCl₂, CHCl=CHCl, chlorobenzene, dichlorobenzene, trichlorobenzene, benzene, toluene, diethyl ether, diisopropyl ether, methyl tert butyl ether, THF, Me-THF, dioxane, dioxolane, acetone, acetonitrile, diethyl ether, diisopropyl ether, methyl tert butyl ether, glyme.

In certain embodiments, the first step synthesis uses a catalyst such as Dimethylformamide (DMF), Dimethylacetamide (DMAC), N-Methyl-2-pyrrolidone (NMP), 1,3-Dimethyl-2-imidazolidinone (DMI), N,N ' -Dimethylpropyleneurea (DMPU), Hexamethylphosphoramide (HMPA), 4-Dimethylaminopyridine (DMAP), pyridine, lutidine, 1,4-diazabicyclo[2.2. 2]octane (DABCO), quinuclidine, triethylamine.

In certain embodiments, the first step synthesis uses a chlorinating agent such as SOCl₂ or (COCl)₂.

In certain embodiments, the temperature of the first step synthesis is comprised between -10°C and 80°C.

In certain embodiments, the duration of the first step synthesis is comprised between 30 minutes and 24h.

In certain embodiments, the amine compound in the second step is aryl amine that may be substituted. In some instances, the substitutions are independently selected from H, F, Cl, Br, CH₃, I, OH, OMe, COOH, COOR, CHO, COCl, COF, CN, CH(COR)₂, CH(CN)₂, OAc, OTMS, OTBS, OC(CH₃)₃, O(CO)OC(CH₃)₃, O(CH₂CH₂O)n, O(CH(CH₃)CH₂O)ₙ, OSO₃H, OSO₃R, OSO₂F, OSO₂Cl, OSO(NR)F, NO₂, NH₂, NHR, NR₂, NHAc, NH(CO)OC(CH₃)₃, NSOF₂, NSO(OR)F, NSO(NR)F, NSO(OR)₂, NSO(NR)₂, NCO, SH, SR, SO₃H, SO₂F, SO₂Cl, SO₂NH₂, SO₂NHR, SO₂NR₂, SO₂(OR) or SO₂NHSO₂R, wherein R is an alkyl compound.

In certain embodiments, the methods of the present technology comprise the second step synthesis is in an aprotic solvent such as DCM, CHCl₃, CCl₄, Cl₂C=CCl₂, CHCl=CHCl, chlorobenzene, dichlorobenzene, trichlorobenzene, benzene, toluene, diethyl ether, diisopropyl ether, methyl tert butyl ether, THF, Me-THF, dioxane, dioxolane, acetone, acetonitrile, diethyl ether, diisopropyl ether, methyl tert butyl ether, glyme.

In certain embodiments, the temperature of the second step synthesis is comprised between -10°C and 80°C.

In certain embodiments, the duration of the second step synthesis is comprised between 30 minutes and 24h.

In certain embodiments, the compound that is suitable to act as a quenching base is a basic compound. In some embodiments, the basic compound may be selected from triethylamine, K₂CO₃, Na₂CO₃, Li₂CO₃, LiOH, Li₂SO₃, Li₃PO₄, lithium acetate, lithium formate, pyridine, DMAP, DABCO, quinuclidine and combinations thereof. In one embodiment, the basic compound is triethylamine.

In certain embodiments, the lithiation step is done with a compound that acts as source of lithium. The source of lithium may be selected from Li₂CO₃, LiOH, Li₂SO₃, Li₃PO₄, lithium acetate, lithium formate. In one embodiment, the lithium source is LiOH.

In certain embodiments, the methods of the present technology comprise the following steps: 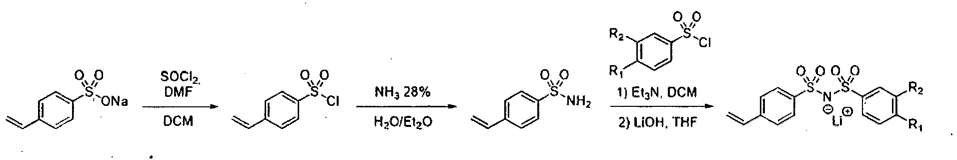 wherein R₁ and R₂ are independently H, F, Cl, Br, CH₃, I, OH, OMe, COOH, COOR, CHO, COCl, COF, CN, CH(COR)₂, CH(CN)₂, OAc, OTMS, OTBS, OC(CH₃)₃, O(CO)OC(CH₃)₃, O(CH₂CH₂O)ₙ, O(CH(CH₃)CH₂O)ₙ, OSO₃H, OSO₃R, OSO₂F, OSO₂Cl, OSO(NR)F, NO₂, NH₂, NHR, NR₂, NHAc, NHTf, NH(CO)OC(CH₃)₃ NSOF₂, NSO(OR)F, NSO(NR)F, NSO(OR)₂, NSO(NR)₂, NCO, SH, SR, SO₃H, SO₂F, SO₂Cl, SO₂NH₂, SO₂NHR, SO₂NR₂, SO₂(OR) or SO₂NHSO₂R, wherein R is an alkyl compound.

In certain embodiments, the methods of the present technology comprise the first step synthesis in an aprotic solvent such as DCM, CHCl₃, CCl₄, Cl₂C=CCl₂, CHCl=CHCl, chlorobenzene, dichlorobenzene, trichlorobenzene, benzene, toluene, diethyl ether, diisopropyl ether, methyl tert butyl ether, THF, Me-THF, dioxane, dioxolane, acetone, acetonitrile, diethyl ether, diisopropyl ether, methyl tert butyl ether, glyme.

In certain embodiments, the first step synthesis uses a catalyst such as DMF, DMAC, NMP, DMI, DMPU, HMPA, DMAP, pyridine, lutidine, DABCO, quinuclidine, triethylamine.

In certain embodiments, the first step synthesis uses a chlorinating agent such as SOCl₂ or (COCl)₂.

In certain embodiments, the temperature of the first step synthesis is comprised between -10°C and 80°C.

In certain embodiments, the duration of the first step synthesis is comprised between 30 minutes and 24h.

In certain embodiments, the source of ammonia in the second step is an aqueous ammonium hydroxide solution of concentration between 0.001-30 %, liquid ammonia, a stream of ammonia gas or ammonium salts.

In certain embodiments, the methods of the present technology comprise the second step synthesis is in an aprotic solvent such as DCM, CHCl₃, CCl₄, Cl₂C=CCl₂, CHCl=CHCl, chlorobenzene, dichlorobenzene, trichlorobenzene, benzene, toluene, diethyl ether, diisopropyl ether, methyl tert butyl ether, THF, Me-THF, dioxane, dioxolane, acetone, acetonitrile, diethyl ether, diisopropyl ether, methyl tert butyl ether, glyme.

In certain embodiments, the temperature of the second step synthesis is comprised between -10°C and 80°C.

In certain embodiments, the duration of the second step synthesis is comprised between 30 minutes and 24h.

In certain embodiments, the methods of the present technology comprise the second step synthesis is in an aprotic solvent such as DCM, CHCl₃, CCl₄, Cl₂C=CCl₂, CHCl=CHCl, chlorobenzene, dichlorobenzene, trichlorobenzene, benzene, toluene, diethyl ether, diisopropyl ether, methyl tert butyl ether, THF, Me-THF, dioxane, dioxolane, acetone, acetonitrile, diethyl ether, diisopropyl ether, methyl tert butyl ether, glyme.

In certain embodiments, the temperature of the second step synthesis is comprised between -10°C and 80°C.

In certain embodiments, the duration of the second step synthesis is comprised between 30 minutes and 24h.

In certain embodiments, the methods of the present technology comprise the third step synthesis is in an aprotic solvent such as DCM, CHCl₃, CCl₄, Cl₂C=CCl₂, CHCl=CHCl, chlorobenzene, dichlorobenzene, trichlorobenzene, benzene, toluene, diethyl ether, diisopropyl ether, methyl tert butyl ether, THF, Me-THF, dioxane, dioxolane, acetone, acetonitrile, diethyl ether, diisopropyl ether, methyl tert butyl ether, glyme. In one embodiment the third step synthesis is done in DCM.

In certain embodiments, the temperature of the third step synthesis is comprised between -10°C and 80°C.

In certain embodiments, the duration of the third step synthesis is comprised between 30 minutes and 24h.

In certain embodiments, the compound that is suitable to act as a quenching base is a basic compound. In some embodiments, the basic compound may be selected from triethylamine, K₂CO₃, Na₂CO₃, Li₂CO₃, LiOH, Li₂SO₃, Li₃PO₄, lithium acetate, lithium formate, pyridine, DMAP, DABCO, quinuclidine and combinations thereof. In one embodiment, the basic compound is triethylamine.

In certain embodiments, the lithiation step is done with a compound that acts as source of lithium. The source of lithium may be selected from Li₂CO₃, LiOH, Li₂SO₃, Li₃PO₄, lithium acetate, lithium formate. In one embodiment, the lithium source is LiOH.

In such embodiments, the lithium single-ion monomer obtained has formula: 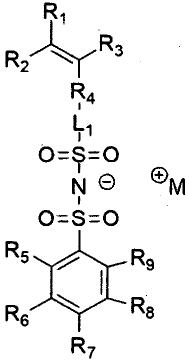
wherein R₁ and R₂ are independently H or F; R₃ is H, F, CN or CH₃; R₄ is an ester group, a phenyl group with L₁ substituted at ortho, para, or meta position, a fully or partially fluorinated phenyl group with L₁ substituted at ortho, para, or meta position, an amide group, a carbonate group, or an ether group; L₁ is a n-alkyl group (e.g., n=1-16), a branched alkyl group (e.g., C₃-C₁₆), an ethylene oxide linker (e.g., C₄ to C₁₆), a cycloalkyl group (e.g., C₄ to C₇), or L₁ is absent;
R₅, R₆, R₇, R₈, R₉ are independently selected from H, F, CN, NO₂, an alkoxy group and an aryl group; and
M is monovalent cation H⁺, Li⁺, Na⁺, K⁺, Rb⁺ or Cs⁺.

In some instances, the ester group is -(C=O)-O- or -O-(C=O)-). In some instances, the amide group is -(C=O)-NH-. In some instances, the carbonate group is (-O-(C=O)-O-). In some instances, the ether group is (-O-).

In some instances, the monovalent cation M is Li⁺.

In other embodiments, the methods of the present technology comprise 1) obtaining a sulfonyl chloride compound from a sulfonate; 2) simultaneously reacting a sulfonyl chloride compound with: i) an aromatic sulfonamide compound; and ii) a compound that is suitable to act as a quenching base to obtain unpurified lithium single-ion monomer; and 3) purifying the unpurified lithium single-ion monomer, as described above. In certain embodiments, the aromatic sulfonamide compound has the formula R_{A}-SO2-NH2, wherein R_{A} is an aryl compound substituted which can have electron-withdrawing group. In some embodiments the electron-withdrawing group is selected from -CN, -NO₂, -F, OMe.

In certain embodiments, the mass yield of the lithium single-ion monomer obtained by the methods of the present technology is between about 60% and about 99%, between about 70% and about 80%, between about 80% and 99%, about 75%, or about 95%.

### EXAMPLES

### Example 1 - Synthesis of 4-styrenesulfonyl chloride (SSCl)

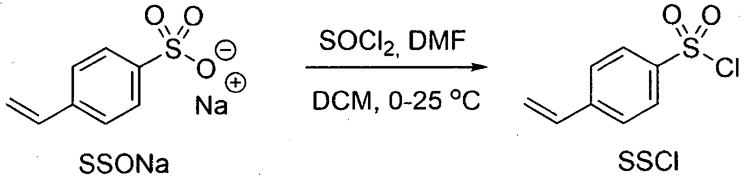

For this synthesis, 22.98 g (111.46 mmol) of sodium 4-styrenesulfonate (SSONa) are weighted and added to a clean, dry 3-necked flask equipped with a magnetic stirrer and an addition funnel. The flask is sealed and air is evacuated by flushing with nitrogen. 100 mL of DCM are added to the flask and the mixture is stirred at 400 rpm. While stirring, 3.43 mL (44.58 mmol, 0.4 eq.) of anhydrous DMF are added and the mixture is cooled by submerging the flask in an ice/water bath. 12.13 mL (167.18 mmol, 1.5 eq.) of thionyl chloride are loaded into the addition funnel and added dropwise to the stirred, cooled mixture. The contents are gradually warmed up to room temperature by stirring in the ice/water bath over a period of 16h. After completion, 100 mL of cold deionised water are added to the flask and the contents are stirred for at least 5 minutes. The phases are then decanted using a separation funnel and the aqueous layer is extracted with 80 mL of DCM. The combined organic phases are washed once with 100 mL of deionised water, then with 100 mL of an aqueous solution of 1M NaHCO3 and finally with 100 mL of deionised water. The organic fraction is dried over anhydrous Na2SO4 then passed through a Whatmann 54 filter paper and the solvent is removed by rotary evaporation. 19.66 g (97.01 mmol, 87.0 %) of a yellow oil are collected and used directly in the next step. **¹H** NMR (CDCl3, 60 MHz): δ 8.06-7.92 (d, 2H), 7.64-7.52 (d, 2H), 7.03-6.58 (dd, 1H), 6.08-5.80 (d, 1H), 5.44-5.29 (d, 1H).

### Example 2 - Synthesis of 4-styrenesulfonamide (SSNH₂)

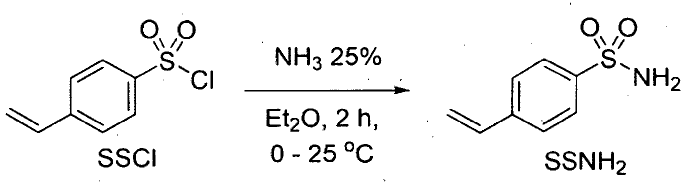

In a clean, dry flask equipped with a magnetic stirrer, 100 mL of an aqueous solution of 25% NH₃ are combined with 75 mL of diethyl ether. The mixture is stirred vigorously and cooled by immersing the flask in a ice/water bath. 4-styrenesulfonyl chloride is weighted in a moisture-free environment and mixed with 50 mL of dry diethyl ether then added directly into the reaction flask and stirred vigorously for at least 2 h. After completion, the mixture is poured on ice and acidified using concentrated HCl until pH = 1 while making sure the temperature does not increase above 25°C. The organic phase is decanted using a separatory funnel and the aqueous phase is then extracted with 3 x 75 mL of ethyl acetate. The combined organic phases are washed with 50 mL of an aqueous solution of 1M NaHCO3 then with 50 mL of deionised water. The organic layer is dried over anhydrous Na2SO4 then passed through a Whatmann 54 filter paper and the solvent is evaporated under reduced pressure. 14.83 g (80.93 mmol, 80.4 %) of a white powder are recovered and stored at room temperature. **¹H NMR** (CDCl3, 60 MHz): δ 7.94-7.80 (d, 2H), 7.65-7.51 (d, 2H), 7.06-6.1 (dd, 1H), 6.05-5.78 (d, 1H), 5.48-5.31 (d, 1H). **HPLC** : 99.9% purity.Synthesis of triethylammonium (phenylsulfonyl)((4-vinylphenyl)sulfonyl)amide (PSSI • Et3NH).

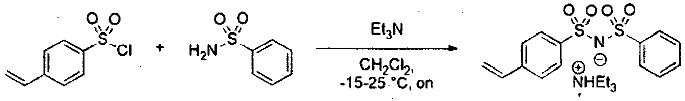

To a mixture of benzenesulfonamide (7.53 g, 47.9 mmol, 0.95 eq.) in anh. CH₂Cl₂ (90.0 mL, 3vol), SSCl (91% in CH₂Cl₂, 11.0 g, 49.4 mmol) is added and it is stirred at rt for 30 min. It is chilled to -15 °C and triethylamine (27.8 mL, 197.6 mmol, 4.0 eq.) is added dropwise. It is slowly warmed to room temperature and stirred at this temperature overnight (12-16 h). H₂O is added and the layers are separated. The organic layer is washed with H₂O (2×), dried (MgSO₄), filtered, MeHQ added and evaporated to dryness (35 °C). To insure the evaporation of triethylamine, MeCN is added and it is coevaporated (2 × 3vol). Finally, MeCN (5vol) is added and the product can be stored as a yellow to brownish solution and is used in the next step without further purification or drying step.

### Example 3 - Synthesis of lithium (phenylsulfonyl)((4-vinylphenyl)sulfonyl)amide (PSSI • Li)

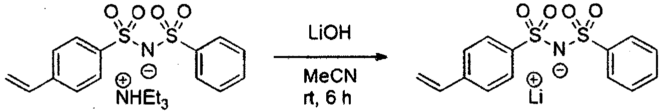

LiOH (1.27 g, 53.0 mmol, 1.5 eq) is added to the solution of PSSI • Et3NH (15.0 g, 35.3 mmol) in MeCN (90 mL) and it is stirred at room temperature for 5.5 h. The suspension is filtered and the filter washed with MeCN. MeHQ is added to the solution and it is evaporated to dryness. The crude product is redissolved in MeCN and precipitated in toluene. It is filtered and the powder dried *in vacuo* at 60°C over night to yield the final product (10.5 g, 11.6 mmol, 64% over two steps) as a white powder. **¹H** NMR (DMSO-*d*₆, 300.13 MHz): δ 7.71-7.57 (m, 4H), 7.50-7.43 (m, 2H), 7.42-7.31 (m, 3H), 6.76 (dd, *J =* 17.7, 10.9 Hz, 1H), 5.90 (dd, *J =* 17.7, 1.0 Hz, 1H), 5.34 (dd, *J =* 10.9, 1.0 Hz, 1H); Purity: 97.9% (HPLC).

### Example 4 - Synthesis of poly(lithium (phenylsulfonyl)((4-vinylphenyl)sulfonyl)amide) (p(PSSI • Li)

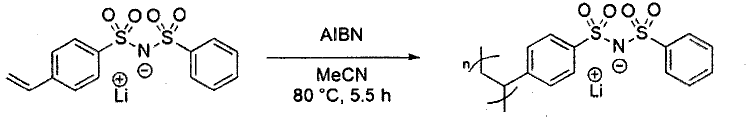

PSSI • Li (10.0 g, 30.4 mmol) is suspended in MeCN (100 mL, purged with nitrogen for 30 min prior to use). AIBN (0.15 g, 0.9 mmol, 3mol%) is added to the mixture and it is purgend with nitrogen for additional 10 min. The mixture is heated to 80 °C and stirred for 5.5 h. Afterwards, it is cooled to room temperature, diluted with MeCN (100 mL) and filtered (cellulose, 20 µm) and the filter washed with MeCN (50 mL). The solids are collected and dried (vacuum, 65 °C, 60 h) to yield the final product as a white fluffy powder (9.15 g, 27.9 mmol, 92%). GPC : Mw = 204,880 g/mol, Mn = 92,292 g/mol, PDI = 2.22.

### Example 5 - Synthesis of triethylammonium (4-fluorophenyl)(4'styrenyl)disulfonimide (FPSSI · Et3NH)

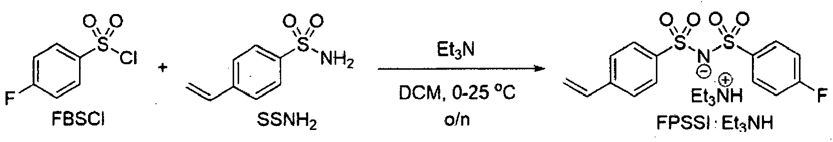

In a clean, dry flask fitted with a magnetic stirrer and an addition funnel, 15.778 g (86.11 mmol) of 4-styrenesulfonamide (SSNH2) are added along with 160 mL of dichloromethane. The flask is sealed and air is evacuated by flushing with a flow of nitrogen, then the mixture is cooled by immersing the flask in an ice/water bath. 20.947 g (107.64 mmol, 1.25 eq.) of 4-fluorobenzenesulfonyl chloride (FBSCl) are weighted in a water-free environment, dissolved with 25 mL of dry DCM then transferred directly into the reaction mixture. The flask used for weighting is washed with an additional 25 mL of DCM which is then transferred to the reactor. 60 mL (430.48 mmol, 5.00 eq.) of freshly distilled triethylamine are loaded into the addition funnel and added dropwise to the cooled and stirred reaction mixture. The flask is slowly warmed to room temperature and stirred over a total of 16h. After completion, 100 mL of deionised water are added to the reactor and stirred with the mixture for at least 5 minutes. Phases are decanted using a separatory funnel and the aqueous layer is extracted with 100 mL of DCM. The combined organic phases are washed with 100 mL of deionised water. 56 mg (0.45 mmol, 0.005 eq.) of mequinol are added to the organic fraction after which the solvent is partially removed under reduced pressure. After evaporation to 20% of the initial volume, 130 mL of THF are added and the solvent is evaporated under reduced pressure. The solvent is evaporated to 20% of the initial volume and diluted again with 130 mL of THF two additional times. The THF solution with a final volume of approximately 200 mL is used directly in the next step without further purification.

### Example 6 - Synthesis of lithium (4-fluorophenyl)(4'styrenyl)disulfonimide (FPSSI · Li)

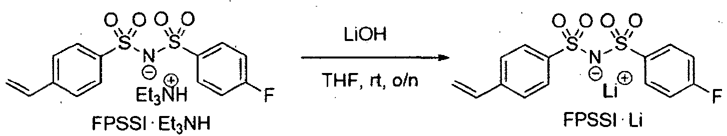

The solution of SFBSI · Et₃NH in THF is added to a clean, dry flask and stirred vigorously with a magnetic stirrer. 4.175 g (174.32 mmol, 2.02 eq.) of finely ground dry LiOH are added and the mixture is stirred for 16 h. The resulting suspension is filtered on a fritt of 10-20 um porosity. 54.5 mg (0.439 mmol, 0.0051 eq.) of mequinol are added to the filtrate and the solvent is evaporated under reduced pressure untill 20% of the initial volume. The residue is mixed with 200 mL of MTBE and the solvent is evaporated under reduced pressure. The residue is dissolved in 150 mL of MeCN and the solvent is partially evaporated under reduced pressure untill 40% of the initial volume, after which 100 mL of toluene are added and the solvents are then evaporated under reduced pressure. The previous step is repeated two additional times. The product is then suspended in 100 mL of DCM, filtered on a fritt of porosity 10-20 um and washed with 2x20 mL of DCM. The product is air dried then further dried under vaccuum for 16h. 22.47g (64.7 mmol, 75.1 % over 2 steps) of white powder are recovered and stored in a sealed container. **¹H** NMR (D₂O, 60 MHz): δ 7.65-7.26 (m, 6 H), 6.97-6.52 (m, 3H), 5.99-5.71 (d, 1H), 5.48-5.31 (d, 1H). ⁷Li **NMR** (D₂O, 60 MHz): δ 0.2061 (s) **¹⁹F NMR** (D₂O, 60 MHz): δ -105.54 (m). **HPLC** : 99.37 % purity.

### Example 7 - Synthesis of p(FPSSI - Li)

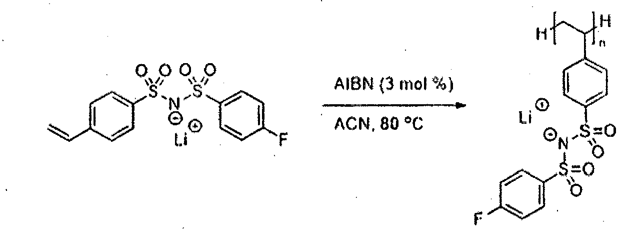

In a clean, dry flask equipped with a magnetic stirrer and a reflux condenser, 1.012 g (2.91 mmol) of FPSSI · Li are added along with 25 mL of acetonitrile. The mixture is degassed by sparging with a flow of dry nitrogen while stirring for at least 30 minutes. AIBN is added to the reaction flask and air is further evacuated with a flow of nitrogen. The flask is then heated to 80 °C and stirred for 6h. The white precipitate is recovered by filtration and dried under vaccuum at 80 °C. 0.8694 g (85.9 %) of a white solid are recovered and stored in a sealed container. **¹H** NMR (D₂O, 60 MHz): δ 7.36 (b), 6.71 (b), 1.14 (b)-. **⁷Li** NMR (D₂O, 60 MHz): δ 0.2155 (b) **¹⁹F** NMR (D₂O, 60 MHz): δ - 104.77 (b). GPC : Mw = 237 084 g/mol, Mn = 72261 g/mol, PDI = 3,281.

### Example 8 - Synthesis of triethylammonium ((3,4-difluorophenyl)sulfonyl)((4-vinylphenyl)sulfonyl)amide (F2PSSI • Et3NH)

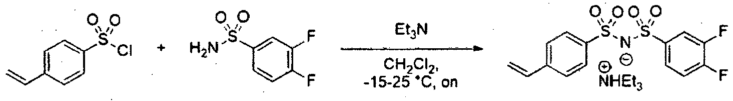

To a mixture of 3,4-difluorobenzenesulfonamide (4.03 g, 20.5 mmol, 0.95 eq.) in anh. CH₂Cl₂ (24.0 mL, 3vol), SSCl (51% in CH₂Cl₂, 8.50 g, 21.5 mmol) is added and it is stirred at rt for 1 h. It is chilled to -15 °C and triethylamine (12.0 mL, 85.2 mmol, 4.0 eq.) is added dropwise. It is slowly warmed to room temperature and stirred at this temperature over night (16 h). H₂O is added, it is stirred for 45 min and the layers are separated. The organic layer is washed with H₂O (2×), dried (MgSO₄), filtered, MeHQ added and evaporated to dryness (35 °C). To insure the evaporation of triethylamine, MeCN is added and it is coevaporated (3vol). Finally, MeCN (40 mL, 5vol) is added and the product can be stored as a yellow to brownish solution and is used in the next step without further purification or drying step. The purity is determined by ¹H NMR and yield calculated to be 8.68 g, 18.8 mmol, 86%. **¹H** NMR (DMSO-*d*₆, 400.16 MHz): δ 7.60-7.57 (m, 2H), 7.53-7.38 (m, 5H), 6.75 (dd, J = 17.7, 11.0 Hz, 1H), 5.90 (dd, J = 17.7, 0.9 Hz, 1H), 5.34 (dd, J = 11.0, 0.9 Hz, 1H), 3.09 (q, J = 7.3 Hz, 6H), 1.17 (t, J = 7.3 Hz, 9H).

### Example 9 - Synthesis of lithium ((3,4-difluorophenyl)sulfonyl)((4-vinylphenyl)sulfonyl)amide (F2PSSI • Li)

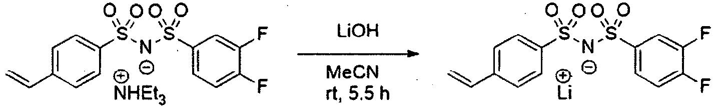

LiOH (0.68 g, 28.3 mmol, 1.5 eq) is added to the solution of F2PSSI • Et3NH (8.68 g, 18.9 mmol) in MeCN (40 mL) and it is stirred at room temperature for 5.5 h. The suspension is filtered and the filter washed with MeCN. MeHQ is added to the solution and it is evaporated to dryness. The crude product is redissolved in MeCN and precipitated in CH₂Cl₂. It is filtered and the powder dried *in vacuo* at 60°C over night to yield the final product (6.3 g, 17.2 mmol, 91%) as a white powder. **¹H NMR** (DMSO-*d*₆, 400.16 MHz): δ 7.60-7.57 (m, 2H), 7.54-7.37 (m, 5H), 6.75 (dd, J = 17.7, 10.9 Hz, 1H), 5.90 (dd, J = 17.7, 0.8 Hz, 1H), 5.35 (dd, J = 10.9, 0.8 Hz, 1H); **¹⁹F NMR** (DMSO-*d*₆, 376.49 MHz): δ -136.3 *(d, J= 21.6* Hz), -138.15 (d, *J =* 21.6 Hz); Purity: 97.9% (HPLC).

### Example 10 - Synthesis of poly(lithium ((3,4-difluorophenyl)sulfonyl)((4-vinylphenyl)sulfonyl)amide) (p(F2PSSI • Li))

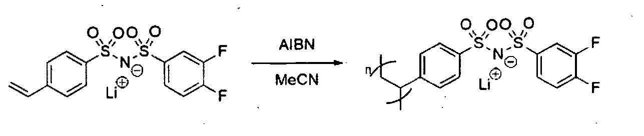

F2SSI • Li (5.0 g, 13.3 mmol) is suspended in MeCN (50 mL, purged with nitrogen fo 30 min prior to use). AIBN (66.7 mg, 398 µmol, 3mol%) is added to the mixture and it is purged with nitrogen for additional 10 min. The mixture is heated to 80̊C and stirred for 6.0 h. Afterwards, it is cooled to room temperature, diluted with MeCN (50 mL) and filtered (cellulose, 20 µm). The filter is washed with MeCN (50 mL). The solids are collected and dried (vacuum, 65̊C, 60h) to yield the final product as a white fluffy powder.

### Example 11 - Synthesis of triethylammonium ((4-nitrophenyl)sulfonyl)((4- vinlyphenyl)sulfonyl)amide (NPSSI•Et3NH)

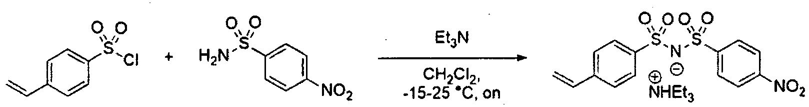

To a mixture of 4-nitrobenzenesulfonamide (4.22 g, 21.4 mmol, 0.99 eq.) in anh. CH₂Cl₂ (24.0 mL, 3vol), SSCl (51% in CH₂Cl₂, 8.51 g, 21.6 mmol) is added and it is stirred at rt for 1 h. It is chilled to -15 °C and triethylamine (12.0 mL, 85.2 mmol, 4.0 eq.) is added dropwise. It is slowly warmed to room temperature and stirred at this temperature over night (16 h). H₂O is added, it is stirred for 4 h and the layers are separated. The organic layer is washed with H₂O (2×), dried (MgSO₄), filtered, MeHQ added and evaporated to dryness (35 °C). To insure the evaporation of triethylamine, MeCN is added and it is coevaporated (3vol). Finally, MeCN (40 mL, 5vol) is added and the product can be stored as a yellow to brownish solution and is used in the next step without further purification or drying step. The purity is determined by ¹H NMR and yield calculated to be 8.96 g, 19.1 mmol, 89%. **¹H** NMR (DMSO-*d*₆, 400.16 MHz): δ 8.24-8.14 (m, 2H), 7.91-7.82 (m, 2H), 7.64-7.56 (m, 2H), 7.49-7.41 (m, 2H), 6.74 (dd, *J =* 17.6, 11.0 Hz, 1H), 5.88 (dd, *J =* 17.6, 1.0 Hz, 1H), 5.34 (dd, *J =* 11.0, 1.0 Hz, 1H), 3.09 (q, *J =* 7.3 Hz, 6H), 1.17 (t, *J =* 7.3 Hz, 9H).

### Example 12 - Synthesis of lithium (4-nitrophenyl)sulfonyl)((4-vinylphenyl)sulfonyl)amide (NPSSI • Li)

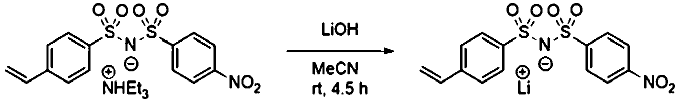

LiOH (0.69 g, 28.6 mmol, 1.5 eq) is added to the solution of NPSSI • Et3NH (8.96 g, 19.1 mmol) in MeCN (40 mL) and it is stirred at room temperature for 4.5 h. The suspension is filtered and the filter washed with MeCN. MeHQ is added to the solution and it is evaporated to dryness. The crude product is redissolved in MeCN and precipitated in CH₂Cl₂. It is filtered and the powder dried *in vacuo* at 60 °C for 2 d to yield the final product (7.0 g, 18.8 mmol, 99%) as a white powder. **¹H** NMR (DMSO-*d*₆, 400.16 MHz): δ 8.22-8.18 (m, 2H), 7.89-7.85 (m, 2H), 7.61-7.44 (m, 2H), 7.46-7.44 (m, 2H), 6.74 (dd, *J =* 17.7, 10.9 Hz, 1H), 5.88 (dd, *J* = 17.7, 0.9 Hz, 1H), 5.34 (dd, *J* = 10.9, 0.9 Hz, 1H); Purity: 99.6% (HPLC).

### Example 13 - Synthesis of poly(lithium ((4-nitrophenyl)sulfonyl)((4-vinylphenyl)sulfonyl)amide) (p(NPSSI • Li))

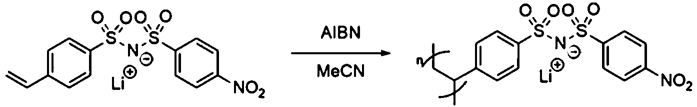

NSSI · Li (5.0 g, 13.3 mmol) is suspended in MeCN (50 mL, purged with nitrogen for 30 min prior to use). AIBN (67.7 mg, 404 µmol, 3mol%) is added to the mixture and it is purgend with nitrogen for additional 10 min. The mixture is heated to 80 °C and stirred for 6.0 h. Afterwards, it is cooled to room temperature, diluted with MeCN (50 mL) and slowly dropped into toluene (350 mL). The precipitate is filtered (cellulose, 20 µm), the solids are collected and dried (vacuum, 65 °C, 60 h) to yield the final product as a white fluffy powder.

It should be appreciated that the present technology is not limited to the particular embodiments described and illustrated herein but includes all modifications and variations falling within the scope of the present technology as defined in the appended claims.

All references cited in this specification, and their references, are incorporated by reference herein in their entirety where appropriate for teachings of additional or alternative details, features, and/or technical background.

## Claims

1. A method for the synthesis of a lithium single-ion perfluoroalkyl and polyfluoroalkyl substances (PFAS)-free monomer, the method comprising simultaneously reacting a sulfonyl chloride compound with:
i) an aromatic sulfonamide compound; and
ii) a compound that is suitable to act as a quenching base;
wherein the simultaneous reaction of sulfonyl chloride with the aromatic sulfonamide compound and the compound that is suitable to act as a quenching base yields the single-ion monomer; wherein the sulfonyl chloride compound is of formula: 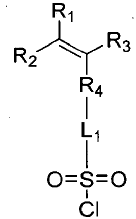
wherein
R₁ and R₂ are each independently H or F;
R₃ is H, F, CN or CH₃;
R₄ is an ester group, a phenyl group with L₁ substituted at ortho, para, or meta position, a fully or partially fluorinated phenyl group with L₁ substituted at ortho, para or meta position, an amide group, a carbonate group, or an ether group; and
L₁ is a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent.

2. The method of claim 1, wherein the aromatic sulfonamide compound has a formula R_{A}-SO2-NH2, wherein R_{A} is a substituted aryl compound.

3. The method of claim 2, wherein the substituted aryl compound comprises an electron withdrawing group selected from -CN, -NO₂, -F, and -OMe.

4. The method of any one of claims 1 to 3, wherein the sulfonate compound is of formula: 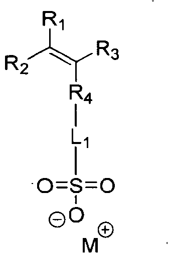 wherein
R₁ and R₂ are each independently H or F;
R₃ is H, F, CN or CH₃;
R₄ is an ester group, a phenyl group with L₁ substituted at ortho, para, or meta position, a fully or partially fluorinated phenyl group with L₁ substituted at ortho, para, or meta position, an amide group, a carbonate group, or an ether group;
L₁ is a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent; and
M is monovalent cation H⁺, Li⁺, Na⁺, K⁺, Rb⁺ or Cs⁺.

5. The method of claim 4, wherein the conversion from sulfonate to sulfonyl chloride is performed using one or more of thionyl chloride and oxalyl chloride.

6. The method of any one of claims 1 to 5, wherein the sulfonyl chloride compound has the formula: 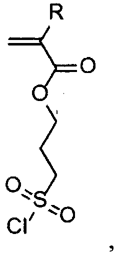 wherein R is H or CH₃, 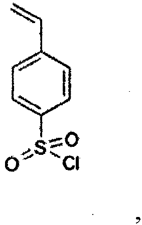 or

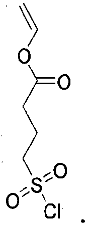

7. The method of claim 6, wherein the sulfonate has the formula: 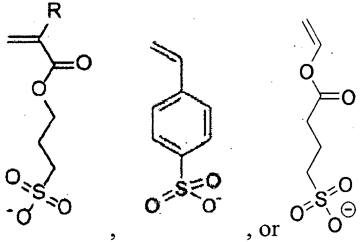 wherein R is H or CH₃.

8. A method for the synthesis of a lithium single-ion perfluoroalkyl and polyfluoroalkyl substances (PFAS)-free monomer, the method comprising simultaneously reacting a sulfonyl chloride compound with:
i) an aromatic sulfonamide compound; and
ii) a compound that is suitable to act as a quenching base;
wherein the simultaneous reaction of sulfonyl chloride with the aromatic sulfonamide compound and the compound that is suitable to act as a quenching base yields the single-ion monomer; wherein the aromatic sulfonamide compound is of formula: 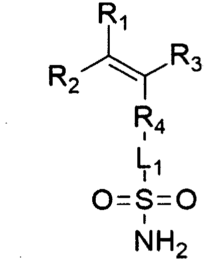
wherein
R₁ and R₂ are each independently H or F;
R₃ is H, F, CN or CH₃;
R₄ is an ester group, a phenyl group with L₁ substituted at ortho, para, or meta position, a fully or partially fluorinated phenyl group with L₁ substituted at ortho, para or meta position, an amide group, a carbonate group, or an ether group; and
L₁ is a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent.

9. The method of claim 8, wherein the sulfonyl chloride compound has a formula R_{A}-SO₂-Cl, wherein R_{A} is a substituted aryl compound.

10. The method of any one of claims 1 to 9, wherein the method comprises the following steps: 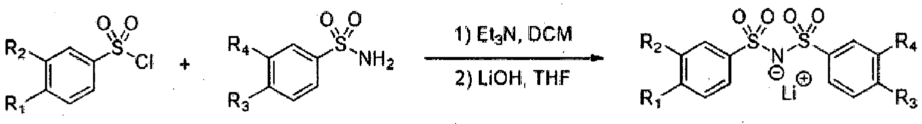 wherein R₁, R₂, R₃ and R₄ are independently H, CH₃, C₂H₃, F, OMe or NO₂.

11. The method of claim 1, wherein the lithium single-ion (PFAS)-free compound is selected from:
i) Poly(lithium (phenylsulfonyl)((4-vinylphenyl)sulfonyl)amide) (PSSI - Li),
ii) Poly(lithium (4-nitrophenyl)sulfonyl)((4-vinylphenyl)sulfonyl)amide) (NPSSI - Li),
iii) Poly(lithium (4-fluorophenyl)sulfonyl)((4-vinylphenyl)sulfonyl)amide) (FPSSI - Li),
iv) Poly(lithium (3,4-difluorophenyl)sulfonyl)((4-vinylphenyl)sulfonyl)amide)(F2PSSI - Li),
v) Poly(lithium (3,4-dinitrophenyl)sulfonyl)((4-vinylphenyl)sulfonyl)amide) (N2PSSI - Li)
vi) Poly(lithium difluorovinyl diphenyl sulfonimide) (PPSVSA-F₂),
vii) Lithium (4-fluorophenyl)(4'-styrenyl)disulfonimide (FPSSI·Li),
viii) p(FPSSi·Li),
ix) Lithium (4-anisyl)(4'-styrenyl)disulfonimide (ASSI·Li),
x) Lithium (4-acetamidophenyl)(4'-styrenyl)disulfonimide (AAPSSI·Li),
xi) Lithium (4-phenyl)(4'-styrenyl)disulfonimide (PSSI-Li),
xii) p(PSSI·Li),
xiii) Lithium (4-nitrophenyl)(4'-styrenyl)disulfonimide (NPSSI·Li),
xiv) p(NPSSI-Li),
xv) Lithium (3,4-difluorophenyl)(4'-styrenyl)disulfonimide (F2PSSI·Li),
xvi) p(F₂PSSI·Li),
xvii) Lithium (4-fluorophenyl) (4'-styrenyl)disulfonimide (FPSSI·Li),
xviii) p(FPSSI·Li), Lithium (4-anisyl)(4'-styrenyl)disulfonimide (ASSI·Li), and
xix) Lithium (4-acetamidophenyl)(4'-styrenyl)disulfonimide (AAPSSI·Li).

12. A method for the synthesis of a lithium single-ion monomer, the method comprising:
1) obtaining a sulfonyl chloride compound from a sulfonate;
2) simultaneously reacting the sulfonyl chloride compound with:
i) an aromatic sulfonamide compound, and
ii) a compound that is suitable to act as a quenching base to obtain unpurified lithium single-ion monomer; and
3) purifying the unpurified lithium single-ion (PFAS)-free monomer.

13. The method of claim 12, wherein the aromatic sulfonamide compound has the formula R_{A}-SO2-NH2, wherein R_{A} is a substituted aryl compound.

14. A lithium single-ion (PFAS)-free monomer having formula: 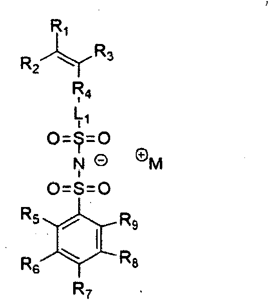 wherein
R₁ and R₂ are independently H or F;
R₃ is H, F, CN or CH₃;
R₄ is an ester group, a phenyl group with L₁ substituted at ortho, para, or meta position, a fully or partially fluorinated phenyl group with L₁ substituted at ortho, para, or meta position, an amide group, a carbonate group, or an ether group;
L₁ can be a n-alkyl group, a fluorinated alkyl group, a branched alkyl group, an ethylene oxide linker, a fluorinated ethylene oxide linker, a cycloalkyl group, a fluorinated cycloalkyl group, or L₁ is absent;
R₅, R₆, R₇, R₈, R₉ are selected from H, F, CN, NO₂, an alkoxy group; and
M is monovalent cation H⁺, Li⁺, Na⁺, K⁺, Rb⁺ or Cs⁺.

15. A lithium single-ion (PFAS)-free compound selected from:
i) Poly(lithium (phenylsulfonyl)((4-vinylphenyl)sulfonyl)amide) (PSSI - Li),
ii) Poly(lithium (4-nitrophenyl)sulfonyl)((4-vinylphenyl)sulfonyl)amide) (NPSSI - Li),
iii) Poly(lithium (4-fluorophenyl)sulfonyl)((4-vinylphenyl)sulfonyl)amide) (FPSSI - Li),
iv) Poly(lithium (3,4-difluorophenyl)sulfonyl)((4-vinylphenyl)sulfonyl)amide)(F2PSSI - Li),
v) Poly(lithium (3,4-dinitrophenyl)sulfonyl)((4-vinylphenyl)sulfonyl)amide) (N2PSSI - Li)
vi) Poly(lithium difluorovinyl diphenyl sulfonimide) (PPSVSA-F₂),
vii) Lithium (4-fluorophenyl)(4'-styrenyl)disulfonimide (FPSSI·Li),
viii) p(FPSSi·Li),
ix) Lithium (4-anisyl)(4'-styrenyl)disulfonimide (ASSI·Li),
x) Lithium (4-acetamidophenyl)(4'-styrenyl)disulfonimide (AAPSSI·Li),
xi) Lithium (4-phenyl)(4'-styrenyl)disulfonimide (PSSI·Li),
xii) p(PSSI·Li),
xiii) Lithium (4-nitrophenyl)(4'-styrenyl)disulfonimide (NPSSI·Li),
xiv) p(NPSSI-Li),
xv) Lithium (3,4-difluorophenyl)(4'-styrenyl)disulfonimide (F2PSSI·Li),
xvi) p(F₂PSSI·Li),
xvii) Lithium (4-fluorophenyl) (4'-styrenyl)disulfonimide (FPSSI·Li),
xviii) p(FPSSI·Li), Lithium (4-anisyl)(4'-styrenyl)disulfonimide (ASSI·Li), and
xix) Lithium (4-acetamidophenyl)(4'-styrenyl)disulfonimide (AAPSSI·Li).
